# EUROPÄISCHE PATENTSCHRIFT

(11) **EP 1 081 129 B1**
(45) Veröffentlichungstag und Bekanntmachung des Hinweises auf die Patenterteilung: **22.10.2003**
(21) Anmeldenummer: 00118126.2
(22) Anmeldetag: 28.08.2000
(51) Int. Cl.: C07C 211/65, C07C 211/63, C07F 9/54

(54) **Stabile (CF3)2N-Salze, ein Verfahren zu deren Herstellung und ihre Verwendung bei der Synthese von Flüssigkristallverbindungen**
Stable (CF3)2N-Salts, a method of preparing them and their use in the synthesis of liquid crystals
Sels d'amine de formule (CF3)2N stables, procédé de préparation des mêmes sels et leurs utilisations avec la préparation des composées de cristaux liquides

(30) Priorität: 01.09.1999 DE 19941566
(43) Veröffentlichungstag der Anmeldung: 07.03.2001
(73) Patentinhaber: MERCK PATENT GmbH, 64293 Darmstadt (DE)
(72) Erfinder: Heider, Udo, Dr., 64560 Riedstadt (DE); Schmidt, Michael, Dr., 64331 Weiterstadt (DE); Sartori, Peter, Prof. Dr., 86919 Utting (DE); Ignatyev, Nikolai, Dr., 47058 Duisburg (DE); Kucheryna, Andrej, 47053 Duisburg (DE)

(56) Entgegenhaltungen:
- DATABASE CHEMABS [Online] CHEMICAL ABSTRACTS SERVICE, COLUMBUS, OHIO, US; GONTAR, A. F. ET AL: "Bis(trifluoromethyl)azanion" retrieved from STN Database accession no. 84:150135 XP002158162 & IZV. AKAD. NAUK SSSR, SER. KHIM. (1976), (1), 212-14 ,
- DESMARTEAU, DARRYL D. ET AL: "Novel ammonium hexafluoroarsenate salts from reaction of bis(trifluoromethyl)amine, trifluoromethoxy(trifluoromethyl)amine, hepatafluoroisopropoxy(trifluoromethyl)ami ne, (trifluoromethyl)fluoroamine, and (pentafluorosulfur)fluoroamine with the strong acid hydrofluoric acid/arsenic fluoride (HF/AsF5)" J. FLUORINE CHEM., Bd. 25, Nr. 3, 1984, Seiten 387-394, XP000980633
- MINKWITZ, R. ET AL: "Contribution to the Chemistry of Bis(trifluoromethyl)amines: Preparation of Bis(trifluoromethyl)ammonium Hexafluorometalates (CF3)2NH2+MF6- (M = As, Sb). Crystal Structure of(CF3)2NH2+AsF6- and Gas-Phase Structures of (CF3)2NX (X = H, Cl)" INORG. CHEM., Bd. 33, Nr. 9, 1994, Seiten 1817-1821, XP000980631
- MINKWITZ, R. ET AL: "Preparation and spectroscopic characterization of trifluoromethyl-substituted amides, phosphides and arsenides, M(CF3)2- (M = N, P, As)" INORG. CHEM. (1989), 28(9), 1627-30 , Bd. 28, Nr. 9, 1989, Seiten 1627-1630, XP000980632
- DATABASE CHEMABS [Online] CHEMICAL ABSTRACTS SERVICE, COLUMBUS, OHIO, US; GONTAR, A. F. ET AL: "Generation and certain reactions of bis(trifluoromethyl)azanion" retrieved from STN Database accession no. 84:121781 CA XP002158163 & IZV. AKAD. NAUK SSSR, SER. KHIM. (1975), (10), 2279-82 , 1975,

## Beschreibung

Die Erfindung betrifft stabile (CF₃)₂N⁻-Salze und ein Verfahren zu deren Herstellung sowie deren Anwendung als Vorstufe für organische Verbindungen.

Lithium-lonen-Batterien gehören zu den aussichtsreichsten Systemen für mobile Anwendungen. Die Einsatzgebiete reichen dabei von hochwertigen Elektronikgeräten (z.B. Mobiltelefone, Camcorder) bis hin zu Batterien für Kraftfahrzeuge mit Elektroantrieb.

Diese Batterien bestehen aus Kathode, Anode, Separator und einem nichtwäßrigen Elektrolyt. Als Kathode werden typischerweise Li(MnMe_{z})₂O₄, Li(CoMe_{z})O₂, Li(CoNiₓMe_{z})O₂ oder andere Lithium-Interkalations und Insertions-Verbindungen verwendet. Anoden können aus Lithium-Metall, Kohlenstoffen, Graphit, graphitischen Kohlenstoffen oder andere Lithium-Interkalations und Insertions-Verbindungen oder Legierungsverbindungen bestehen. Als Elektrolyt werden Lösungen mit Lithiumsalzen wie LiPF₆, LiBF₄, LiClO₄, LiAsF₆, LiCF₃SO₃, LiN(CF₃SO₂)₂ oder LiC(CF₃SO₂)₃ und deren Mischungen in aprotischen Lösungsmitteln verwendet.

Auf der Suche nach neuen Salzen für die Anwendung in Batterien wurde die Gruppe der Bis(trifluoromethyl)amine als geeignet eingestuft.

Cäsiumbis(trifluoromethyl)amid ist eines der ersten in der Literatur beschriebenen Salze aus der Gruppe der Bis(trifluoromethyl)amine. Dieses Salz kann durch einleiten von Perfluoro(2-azapropan) in eine Suspension aus Cäsiumfluorid und trocknem Acetonitril dargestellt werden. Minkwitz (Inorg. Chem., 28 (1989), 1627-1630) isoliert Cs⁺⁻N(CF₃)₂ aber nicht (C₆H₅)₄As⁺ (CF₃)₂N⁻ und (C₂H₅)₄N⁺ (CF₃)₂N⁻.

In der europäischen Anmeldung 99101982 wird eine neue Methode zur Darstellung des (CF₃)₂N⁻ Anions mit einem anorganischen Kation dargelegt. Die Salze sind nur in Lösungen stabil und müssen daher direkt eingesetzt werden.

Gontar, A.F. et al. (IZV. Akad. Nauk SSSR, Ser. Khim. (1976), (1), 212-14) beschreiben die Verbindung Et₃NH⁺ [(CF₃)₂N]⁻ sowie deren Verwendung zur Einführung der Gruppe (CF₃)₂N. Diese wird als stabiles 1:1-Adukt aus (CF₃)₂NH und Triethylamin ((C₂H₅)₃N) in einer Säure-Base-Gleichgewichts-Reaktion gebildet.

Aufgabe der vorliegenden Erfindung ist es deshalb, stabile (CF₃)₂N⁻-Salze und ein Verfahren zu deren Synthese zur Verfügung zu stellen.

Die erfindungsgemäße Aufgabe wird gelöst durch Verbindungen der allgemeinen Formel

[(R¹[CR²R³]ₖ)_{y}Kt]^{+ -}N(CF₃)₂ (I)

wobei
- Kt: N, P, As, Sb, S, Se
R¹, R² und R³ gleich oder verschieden
Halogen, substituiertes und/oder unsubstituiertes Alkyl CₙH₂ₙ₊₁, substituiertes und/oder unsubstituiertes Alkenyl mit 1-18 Kohlenstoffatomen und einer oder mehreren Doppelbindungen, substituiertes und/oder unsubstituiertes Alkinyl mit 1-18 Kohlenstoffatomen und einer oder mehreren Dreifachbindungen, substituiertes und/oder unsubstituiertes Cycloalkyl CₘH₂ₘ₋₁, ein- oder mehrfach substituiertes und/oder unsubstituiertes Phenyl, substituiertes und/oder unsubstituiertes Heteroaryl,
die an Kt gebundenen Gruppen können gleich oder verschieden sein
mit
n 1-18
m 3-7
k 0, 1-6
y 1-4
bedeuten.

Die Verbindungen können als Reagenzien zur Einführung von N(CF₃)₂-Gruppen in organische Substanzen eingesetzt werden. So können beispielsweise fluorinierte Lösungsmittel für sekundäre und primäre Batterien synthetisiert werden.

Überraschend wurde eine weitere Möglichkeit der Anwendung dieser Salze gefunden. Aufgrund ihrer Struktur sind die erfindungsgemäßen Salze als Vorstufen für die Herstellung von Flüssigkristallverbindungen von Interesse.

Es wurde gefunden, daß die einfache Synthese der komplexen Verbindungen unter milden Bedingungen durchgeführt werden kann. Die Salze werden mit hohen Ausbeuten isoliert.

Überraschend wurde gefunden, daß die erfindungsgemäßen Salze stabil sind. Sie können bei Raumtemperatur isoliert und gelagert werden.

Nachfolgend wird ein allgemeines Beispiel der Erfindung näher erläutert.

Verbindungen der allgemeinen Formel

D^{+ -}N(CF₃)₂ (II)

mit D⁺ ausgewählt aus der Gruppe der Alkalimetalle werden nach dem in der europäischen Patentanmeldung Nr.: 99101982 beschriebenen Verfahren hergestellt. Außerdem sind als Lieferant der N(CF₃)₂-Gruppe Verbindungen der allgemeinen Formel

GN(CF₃)₂ (IV)

mit G ausgewählt aus der Gruppe der fluorierten Sulfonamide und fluorierten Acylamide geeignet.

Gelöst in einem geeigneten polaren organischen Lösungsmittel aus der Gruppe Acetonitril, Diethoxyethan und Dimethylformamid wird eine Verbindung der allgemeinen Formel (II) bzw. (IV) vorgelegt. Bei Temperaturen zwischen -40 °C und 80 °C, bevorzugt bei Raumtemperatur, wird ein Salz der allgemeinen Formel

[(R¹[CR²R³]ₖ)_{y}Kt]^{+ -}E (III)

wobei
- Kt=: N, P, As, Sb, S, Se
R¹, R² und R³
gleich oder verschieden
Halogen, substituiertes und/oder unsubstituiertes Alkyl CₙH₂ₙ₊₁, substituiertes und/oder unsubstituiertes Alkenyl mit 1-18 Kohlenstoffatomen und einer oder mehreren Doppelbindungen, substituiertes und/oder unsubstituiertes Alkinyl mit 1-18 Kohlenstoffatomen und einer oder mehreren Dreifachbindungen, substituiertes und/oder unsubstituiertes Cycloalkyl CₘH₂ₘ₋₁, ein- oder mehrfach substituiertes und/oder unsubstituiertes Phenyl, substituiertes und/oder unsubstituiertes Heteroaryl,
die an Kt gebundenen Gruppen können gleich oder verschieden sein
mit
n= 1-18
m= 3-7
k= 0, 1-6
y= 1-4
und
- ⁻E=: F⁻, Cl⁻, Br⁻, J⁻, BF₄⁻, ClO₄⁻, AsF₆⁻, SbF₆⁻ oder PF₆⁻
bedeuten, in äquimolaren Mengen zugegeben.

Es können flüchtige Nebenprodukte entstehen, die durch Anlegen eines Vakuums entfernt werden. Zumeist entstehen jedoch in diesen Lösungsmitteln unlösliche Salze als Nebenprodukt, die abfiltriert werden.

Das Lösungsmittel wird unter reduziertem Druck entfernt. Die Reaktionsprodukte können mit Ausbeuten über 80% gewonnen werden. Die meisten Salze sind bei Raumtemperatur stabil und zersetzen sich beim Schmelzen nicht.

Die erfindungsgemäßen Verbindungen können in Elektrolyten mit herkömmlichen Leitsalzen verwendet werden. Geeignet sind z.B. Elektrolyte mit Leitsalzen ausgewählt aus der Gruppe LiPF₆, LiBF₄, LiClO₄, LiAsF₆, LiCF₃SO₃, LiN(CF₃SO₂)₂ oder LiC(CF₃SO₂)₃ und deren Mischungen. Die Elektrolyte können auch organische Isocyanate (DE 199 44 603) zur Herabsetzung des Wassergehaltes enthalten. Ebenso können die Elektrolyte organische Alkalisalze (DE 199 10 968) als Additiv enthalten. Geeignet sind Alkaliborate der allgemeinen Formel

Li⁺ B⁻(OR¹)ₘ(OR²)ₚ

worin,
- m und p: 0, 1, 2, 3 oder 4 mit m+p=4 und
- R¹ und R²: gleich oder verschieden sind,
gegebenenfalls durch eine Einfach- oder Doppelbindung direkt miteinander verbunden sind,
jeweils einzeln oder gemeinsam die Bedeutung eines aromatischen oder aliphatischen Carbon-, Dicarbon- oder Sulfonsäurerestes haben, oder
jeweils einzeln oder gemeinsam die Bedeutung eines aromatischen Rings aus der Gruppe Phenyl, Naphthyl, Anthracenyl oder Phenanthrenyl, der unsubstituiert oder ein- bis vierfach durch A oder Hal substituiert sein kann, haben oder
jeweils einzeln oder gemeinsam die Bedeutung eines heterocyclischen aromatischen Rings aus der Gruppe Pyridyl, Pyrazyl oder Bipyridyl, der unsubstituiert oder ein- bis dreifach durch A oder Hal substituiert sein kann, haben oder
jeweils einzeln oder gemeinsam die Bedeutung einer aromatischen Hydroxysäure aus der Gruppe aromatischer Hydroxy-Carbonsäuren oder aromatischer Hydroxy-Sulfonsäuren, der unsubstituiert oder einbis vierfach durch A oder Hal substituiert sein kann,
haben und
- Hal: F, Cl oder Br
und
- A: Alkyl mit 1 bis 6 C-Atomen, das ein- bis dreifach halogeniert
sein kann, bedeuten. Ebenso geeignet sind Alkalialkoholate der allgemeinen Formel

Li⁺ OR⁻

worin R
die Bedeutung eines aromatischen oder aliphatischen Carbon-, Dicarbon- oder Sulfonsäurerestes hat, oder
die Bedeutung eines aromatischen Rings aus der Gruppe Phenyl, Naphthyl, Anthracenyl oder Phenanthrenyl, der unsubstituiert oder ein- bis vierfach durch A oder Hal substituiert sein kann, hat oder
die Bedeutung eines heterocyclischen aromatischen Rings aus der Gruppe Pyridyl, Pyrazyl oder Bipyridyl, der unsubstituiert oder ein- bis dreifach durch A oder Hal substituiert sein kann, hat oder
die Bedeutung einer aromatischen Hydroxysäure aus der Gruppe aromatischer Hydroxy-Carbonsäuren oder aromatischer Hydroxy-Sulfonsäuren, der unsubstituiert oder ein- bis vierfach durch A oder Hal substituiert sein kann,
hat und
- Hal: F, Cl, oder Br,
und
A Alkyl mit 1 bis 6 C-Atomen, das ein- bis dreifach halogeniert sein kann.

Auch Lithiumkomplexsalze der Formel 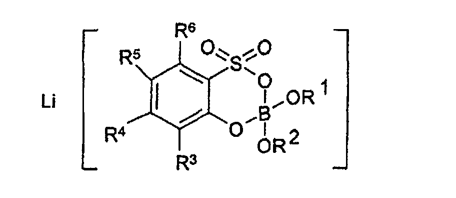 wobei
R¹ und R² gleich oder verschieden sind, gegebenenfalls durch eine Einfach- oder Doppelbildung direkt miteinander verbunden sind, jeweils einzeln oder gemeinsam die Bedeutung eines aromatischen Rings aus der Gruppe Phenyl, Naphthyl, Anthracenyl oder Phenanthrenyl, der unsubstituiert oder ein- bis sechsfach durch Alkyl (C₁ bis C₆), Alkoxygruppen (C₁ bis C₆) oder Halogen (F, Cl, Br) substituiert sein kann, haben,
oder jeweils einzeln oder gemeinsam die Bedeutung eines aromatischen heterozyklischen Rings aus der Gruppe Pyridyl, Pyrazyl oder Pyrimidyl, der unsubstituiert oder ein- bis vierfach durch Alkyl (C₁ bis C₆), Alkoxygruppen (C₁ bis C₆) oder Halogen (F, Cl, Br) substituiert sein kann, haben,
oder jeweils einzeln oder gemeinsam die Bedeutung eines aromatischen Rings aus der Gruppe Hydroxylbenzoecarboxyl, Hydroxylnaphthalincarboxyl, Hydroxylbenzoesulfonyl und Hydroxylnaphthalinsulfonyl, der unsubstituiert oder ein- bis vierfach durch Alkyl (C₁ bis C₆), Alkoxygruppen (C₁ bis C₆) oder Halogen (F, Cl, Br) substituiert sein kann, haben,
R³-R⁶ können jeweils einzeln oder paarweise, gegebenenfalls durch eine Einfach- oder Doppelbindung direkt miteinander verbunden, folgende Bedeutung haben:
1. Alkyl (C₁ bis C₆),Alkyloxy (C₁ bis C₆) oder Halogen (F, Cl, Br)
2. ein aromatischer Ring aus den Gruppen
Phenyl, Naphthyl, Anthracenyl oder Phenanthrenyl, der unsubstituiert oder ein- bis sechsfach durch Alkyl (C₁ bis C₆), Alkoxygruppen (C₁ bis C₆) oder Halogen (F, Cl, Br) substituiert sein kann,
Pyridyl, Pyrazyl oder Pyrimidyl, der unsubstituiert oder ein- bis vierfach durch Alkyl (C₁ bis C₆), Alkoxygruppen (C₁ bis C₆) oder Halogen (F, Cl, Br) substituiert sein kann,
die über folgendes Verfahren (DE 199 32 317) dargestellt werden
a) 3-, 4-, 5-, 6-substituiertes Phenol in einem geeigneten Lösungsmittel mit Chlorsulfonsäure versetzt wird,
b) das Zwischenprodukt aus a) mit Chlortrimethylsilan umgesetzt, filtriert und fraktioniert destilliert wird,
c) das Zwischenprodukt aus b) mit Lithiumtetramethanolat-borat(1-), in einem geeigneten Lösungsmittel umgesetzt und daraus das Endprodukt isoliert wird, können im Elektrolyten enthalten sein.

Aber auch Elektrolyte enthaltend Verbindungen der allgemeinen Formel (DE 199 53 638)

X-(CYZ)ₘ-SO₂N(CR¹R²R³)₂

mit
X H, F, Cl, CₙF₂ₙ₊₁, CₙF₂ₙ₋₁, (SO₂)ₖN(CR¹R²R³)₂
Y H, F, Cl
Z H, F, Cl
R¹, R², R³ H und/oder Alkyl, Fluoralkyl, Cycloalkyl
m 0-9 und falls X=H, m≠0
n 1-9
k 0, falls m=0 und k=1, falls m=1-9,
hergestellt durch die Umsetzung von teil- oder perfluorierten Alkysulfonylfluoriden mit Dimethylamin in organischen Lösungsmitteln sowie Komplexsalze der allgemeinen Formel (DE 199 51 804)

M^{x+}[EZ]^{y-}_{x/y}

worin bedeuten:
- x,y: 1, 2, 3, 4, 5, 6
- M^{x+}: ein Metallion
- E: eine Lewis-Säure, ausgewählt aus der Gruppe
BR¹R²R³, AlR¹R²R³, PR¹R²R³R⁴R⁵, AsR¹R²R³R⁴R⁵, VR¹R²R³R⁴R⁵, R¹ bis R⁵ gleich oder verschieden, gegebenenfalls durch eine Einfach- oder Doppelbildung direkt miteinander verbunden sind, jeweils einzeln oder gemeinsam die Bedeutung
eines Halogens (F, Cl, Br),
eines Alkyl- oder Alkoxyrestes (C₁ bis C₈) der teilweise oder vollständig durch F, Cl, Br substituiert sein kann,
eines, gegebenenfalls über Sauerstoff gebundenen aromatischen Rings aus der Gruppe Phenyl, Naphthyl, Anthracenyl oder Phenanthrenyl, der unsubstituiert oder ein- bis sechsfach durch Alkyl (C₁ bis C₈) oder F, Cl, Br substituiert sein kann
eines, gegebenenfalls über Sauerstoff gebundenen aromatischen heterocyclischen Rings aus der Gruppe Pyridyl, Pyrazyl oder Pyrimidyl, der unsubstituiert oder ein- bis vierfach durch Alkyl (C₁ bis C₈) oder F, Cl, Br substituiert sein kann, haben können und
Z OR⁶, NR⁶R⁷, CR⁶R⁷R⁸, OSO₂R⁶, N(SO₂R⁶)(SO₂R⁷), C(SO₂R⁶)(SO₂R⁷)(SO₂R⁸),
OCOR⁶, wobei
R⁶ bis R⁸ gleich oder verschieden sind, gegebenenfalls durch eine Einfach- oder Doppelbindung direkt miteinander verbunden sind, jeweils einzeln oder gemeinsam die Bedeutung
eines Wasserstoffs oder die Bedeutung wie R¹ bis R⁵ haben, hergestellt durch Umsetzung von einem entsprechenden Bor- oder Phosphor-Lewis-Säure-Solvenz-Adukt mit einem Lithium- oder Tetraalkylammonium-lmid, -Methanid oder -Triflat, können verwendet werden.

Auch Boratsalze (DE 199 59 722) der allgemeinen Formel 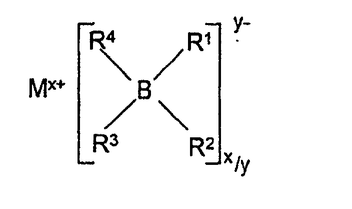 worin bedeuten:
M ein Metallion oder Tetraalkylammoniumion
x,y 1, 2, 3, 4, 5 oder 6
R¹ bis R⁴ gleich oder verschieden, gegebenenfalls durch eine Einfach- oder Doppelbindung direkt miteinander verbundener Alkoxy- oder Carboxyreste (C₁-C₈) können enthalten sein. Hergestellt werden diese Boratsalze durch Umsetzung von Lithiumtetraalkoholatborat oder einem 1:1 Gemisch aus Lithiumalkoholat mit einem Borsäureester in einem aprotischen Lösungsmittel mit einer geeigneten Hydroxyl- oder Carboxylverbindung im Verhältnis 2:1 oder 4:1.

Die erfindungsgemäßen Verbindungen können auch in Elektrolyte eingesetzt werden, die Lithiumfluoralkylphosphate der allgemeinen Formel (I) enthalten,

Li⁺[PFₓ(C_{y}F_{2y+1-z}H_{z})₆₋ₓ]⁻ (I)

worin
1≤x≤5
3≤y≤8
0≤z≤2y+1
bedeuten und die Liganden (C_{y}F_{2y+1-z}H_{z}) gleich oder verschieden sein können, wobei die Verbindungen der allgemeinen Formel (I'),

Li⁺[PFₐ(CH_{b}F_{c}(CF₃)_{d})ₑ]⁻ (I')

in der a eine ganze Zahl von 2 bis 5, b = 0 oder 1, c = 0 oder 1, d = 2 und
e eine ganze Zahl von 1 bis 4 bedeuten, mit den Bedingungen, daß b und c nicht gleichzeitig jeweils = 0 bedeuten und die Summe aus a + e gleich 6 ist und die Liganden (CH_{b}F_{c}(CF₃)_{d}) gleich oder verschieden sein können, ausgenommen sind (DE 100 089 55). Das Verfahren zur Herstellung von Lithiumfluoralkylphosphaten der allgemeinen Formel (I) ist dadurch gekennzeichnet, daß wenigstens eine Verbindung der allgemeinen Formel

HₘP(CₙH₂ₙ₊₁)₃₋ₘ (III),

OP(CₙH₂ₙ₊₁)₃ (IV),

ClₘP(CₙH₂ₙ₊₁)₃₋ₘ (V),

FₘP(CₙH₂ₙ₊₁)₃₋ₘ (VI),

ClₒP(CₙH₂ₙ₊₁)₅₋ₒ (VII),

FₒP(CₙH₂ₙ₊₁)₅₋ₒ (VIII),

in denen jeweils
0 < m <2, 3 < n < 8 und 0 < o < 4 bedeutet
durch Elektrolyse in Fluorwasserstoff fluoriert wird, das so erhaltene Gemisch der Fluorierungsprodukte durch Extraktion, Phasentrennung und/oder Destillation aufgetrennt wird, und das so erhaltene fluorierte Alkylphosphoran in einem aprotischen Lösungsmittel oder Lösungsmittelgemisch unter Feuchtigkeitsausschluß mit Lithiumfluorid umgesetzt wird, und das so erhaltene Salz der allgemeinen Formel (I) nach den üblichen Methoden gereinigt und isoliert wird.

Die erfindungsgemäßen Verbindungen können auch in Elektrolyten eingesetzt werden, die Salze der Formel

Li[P(OR¹)ₐ(OR²)_{b}(OR³)_{c}(OR⁴)_{d}Fₑ]

worin 0 < a+b+c+d ≤ 5 und a+b+c+d+e=6 gilt, und R¹ bis R⁴ unabhängig voneinander Alkyl-, Aryl- oder Heteroarylreste sind, wobei mindestens zwei von R¹ bis R⁴ durch eine Einfach- oder Doppelbindung direkt miteinander verbunden sein können, enthalten (DE 100 16801). Dargestellt werden die Verbindungen durch Umsetzung von Phosphor (V)-Verbindungen der allgemeinen Formel

P(OR¹)ₐ(OR²)_{b}(OR³)_{c}(OR⁴)_{d}Fₑ

worin 0 < a+b+c+d ≤ 5 und a+b+c+d+e=5 gilt, und R¹ bis R⁴ die oben angegebenen Bedeutungen haben mit Lithiumfluorid in Gegenwart eines organischen Lösungsmittels.

Die erfindungsgemäßen Verbindungen können in Elektrolyte für elektrochemische Zellen eingesetzt werden, die Anodenmaterial, bestehend aus beschichteten Metallkernen, ausgewählt aus der Gruppe Sb, Bi, Cd, In, Pb, Ga und Zinn oder deren Legierungen, enthalten (DE 100 16 024). Das Verfahren zur Herstellung dieses Anodenmaterials ist dadurch gekennzeichnet, daß
a) eine Suspension oder ein Sol des Metall- oder Legierungskerns in Urotropin hergestellt wird,
b) die Suspension mit Kohlenwasserstoffen mit C₅-C₁₂ emulgiert werden,
c) die Emulsion auf die Metall- oder Legierungskerne aufgefällt werden und
d) durch Temperung des Systems die Metallhydroxide bzw. -oxihydroxide in das entsprechende Oxid übergeführt werden.

Die erfindungsgemäßen Verbindungen können auch in Elektrolyte für elektrochemische Zellen eingesetzt werden, mit Kathoden aus gängigen Lithium-Interkalations und Insertionsverbindungen aber auch mit Kathodenmaterialien, die aus Lithium-Mischoxid-Partikel bestehen, die mit einem oder mehreren Metalloxiden (DE 199 22 522) beschichtet sind, indem die Partikel in einem organischen Lösungsmittel suspendiert werden, die Suspension mit einer Lösung einer hydrolisierbaren Metallverbindung und einer Hydrolyselösung versetzt und danach die beschichteten Partikel abfiltriert, getrocknet und gegebenenfalls calciniert werden. Sie können auch aus Lithium-Mischoxid-Partikel bestehen, die mit einem oder mehreren Polymeren (DE 199 46 066) beschichtet sind, erhalten durch ein Verfahren, bei dem die Partikel in einem Lösungsmittel suspendiert werden und anschließend die beschichteten Partikel abfiltriert, getrocknet und gegebenenfalls calciniert werden. Ebenso können die erfindungsgemäßen Verbindungen in Systemen mit Kathoden eingesetzt werden, die aus Lithium-Mischoxid-Partikeln bestehen, die mit Alkalimetallverbindungen und Metalloxiden ein- oder mehrfach beschichtet sind (DE 100 14 884). Das Verfahren zur Herstellung dieser Materialien ist dadurch gekennzeichnet, daß die Partikel in einem organischen Lösungsmittel suspendiert werden, eine Alkalimetallsalzverbindung suspendiert in einem organischen Lösungsmittel zugegeben wird, Metalloxide gelöst in einem organischen Lösungsmittel zugegeben werden, die Suspension mit einer Hydrolyselösung versetzt wird und anschließend die beschichteten Partikel abfiltriert, getrocknet und calciniert werden.
Die Reaktionsprodukte der allgemeinen Formel (I) werden auch als N(CF₃)₂-Gruppen-Lieferant in verschieden Reagenzien eingesetzt. So können sie als Vorstufe für Flüssigkristalle genutzt werden.

Zu einer erfindungsgemäß hergestellten Verbindung der Formel (I), die in einem geeigneten Lösungsmittel gelöst wird, wird in äquimolaren Mengen eine Verbindung aus der Gruppe der Alkylhalogenacetate, vorzugsweise Ethylbromacetat, gegeben. Das Gemisch wird 1 bis 4 Stunden, vorzugsweise 2 Stunden, unter Rückfluß erhitzt. Nach der Zugabe von Wasser wird die organische Phase mit geeigneten organischen Lösungsmitteln extrahiert. Der Extrakt wird getrocknet und anschließend das Lösungsmittel abdestilliert.

Die nachfolgenden Beispiele sollen die Erfindung näher erläutern, ohne sie jedoch zu beschränken.

### Beispiele

### Beispiel 1

Eine Lösung von 4.63 g (19.5 mmol) Rb⁺⁻N(CF₃)₂, die aus 2.04 g (19.5 mmol) RbF und 5.56 g(19.5 mmol) CF₃SO₂N(CF₃)₂ in 20 cm³ trocknem Acetonitril hergestellt wurde, wird bei Raumtemperatur unter Rühren zu einer Lösung aus 6.40 g (19.4 mmol) (C₄H₉)₄N⁺BF₄⁻ in 5 cm³ trocknem Acetonitril gegeben. Das ausfallende RbBF₄ wird abfiltriert und mit trocknem Acetonitril gewaschen. Nach der Abtrennung des Lösungsmittels im Vakuum werden 7.5 g weißes Pulver isoliert.

Die Ausbeute an (C₄H₉)₄N⁺⁻N(CF₃)₂ beträgt 98%.

| Analyse: | | |
|---|---|---|
| | gefunden: | berechnet: |
| C | 54.67 | 54.80 |
| H | 9.56 | 9.20 |
| F | 28.70 | 28.90 |
| N | 7.15 | 7.10 |

¹⁹F-NMR (CCl₃F): -38.32 s (solvent: CH₃CN), -37.66 s (solvent: CD₂Cl₂); Schmelzpunkt: 123-125°C

### Beispiel 2

Eine Lösung aus 0.568 g (2.39 mmol) Rb⁺⁻N(CF₃)₂, welche aus 0.25 g (2.39 mmol) RbF und 0.69 g (2.39 mmol) CF₃SO₂N(CF₃)₂ in 2 cm³ trocknem Acetonitril hergestellt wurde, wird bei Raumtemperatur zu einer Lösung aus 0.66 g (2.37 mmol) (C₄H₉)₄N⁺Cl⁻ in 1 cm³ trocknem Acetonitril gegeben. Das ausgefallene RbCI wird abfiltriert und mit trocknem Acetonitril gewaschen. Nach dem Abdestillieren des Lösungsmittels im Vakuum werden 0.77 g eines weißen Pulver isoliert. Die Ausbeute an (C₄H₉)₄N⁺⁻N(CF₃)₂ beträgt 82.2%.
Das ¹⁹F-NMR-Spektrum ist identisch mit dem aus Beispiel 1.

### Beispiel 3

Eine Lösung von 0.62 g (2.61 mmol) Rb⁺⁻N(CF₃)₂, die aus 0.273 g (2.61 mmol) RbF und 0.75 g (2.63 mmol) CF₃SO₂N(CF₃)₂ in 2 cm³ trocknem Acetonitril hergestellt wurde, wird bei Raumtemperatur zu einer Lösung aus 0.883 g (2.60 mmol) (C₄H₉)₄P⁺Br⁻ in 1 cm³ trocknem Acetonitril gegeben. Das ausfallende RbBr wird abfiltriert und mit trocknem Acetonitril gewaschen. Nach dem abdestillieren des Lösungsmittels im Vakuum werden 0.97 g weißes Pulver werden isoliert. Die Ausbeute an (C₄H₉)₄P⁺⁻N(CF₃)₂ beträgt 90.7%.
¹⁹F-NMR (CCl₃F): -36.49 s (solvent: CH₃CN); Schmelzpunkt: 85-86°C

### Beispiel 4

Zu einer Lösung aus 0.522 g (2.20 mmol) Rb⁺⁻N(CF₃)₂, welche aus 0.23 g (2.20 mmol) RbF und 0.63 g (2.21 mmol) CF₃SO₂N(CF₃)₂ in 3 cm³ trocknem Acetonitril hergestellt wird, wird eine Suspension aus 0.84 g (2.16 mmol) Ph₃(PhCH₂)P⁺Cl⁻ in 1 cm³ trocknem Acetonitril gegeben und 10 min bei Raumtemperatur gerührt. Das ausfallende RbCI wird abfiltriert und mit trocknem Acetonitril gewaschen. Nach dem abdestillieren des Lösungsmittels im Vakuum werden 0.96 g weißes Pulver isoliert. Die Ausbeute an Ph₃(PhCH₂)P⁺⁻N(CF₃)₂ beträgt 88.0%.
¹⁹F-NMR (CCl₃F): -36.66 s (solvent: CH₃CN);
Schmelzpunkt: 114-115°C

### Beispiel 5

Zu einer Lösung aus 0.017 g (0.18 mmol) (CH₃)₄N⁺F⁻ in 0.5 cm³ trocknem Dichlormethan werden 0.052 g (0.18 mmol) CF₃SO₂N(CF₃)₂ bei -40 °C gegeben. Die Reaktionslösung wird auf Raumtemperatur erwärmt, mit der gleichen Menge trocknem Acetonitril verdünnt und untersucht mit ¹⁹F-NMR-Spektroskopie. Das gefundene Signal gehört zum Salz (CH₃)₄N⁺⁻N(CF₃)₂. Nach der Abdestillation des Lösungsmittels unter trockner Argonatmosphäre können 0.037 g eines weißen, stark hygroskopischen Materials isoliert werden. Die Ausbeute beträgt 90.2%.
¹⁹F-NMR (CCl₃F): -40.8 s; Schmelzpunkt: 120-125°C

### Beispiel 6

Zu einer Lösung aus 0.837 g (2.12 mmol) (C₄H₉)₄N⁺⁻N(CF₃)₂ in 2 cm³ trocknem Dichlormethan werden 0.271 g (1.62 mmol) BrCH₂COOC₂H₅ gegeben. Die Mischung wird 2 Stunden unter Rückfluß gekocht. Nach der Zugabe von Wasser wird die organische Phase dreimal mit je 10 cm³ Dichlormethan extrahiert. Der Extrakt wird über MgSO₄ getrocknet und das Lösungsmittel abdestilliert. Das erhaltene Produkt, (CF₃)₂NCH₂COOC₂H₅ wird im GC identifiziert. Die Ausbeute beträgt 93.3%.

## Patentansprüche

1. Verbindungen der allgemeinen Formel
[(R¹[CR²R³]ₖ)_{y}Kt]^{+ -}N(CF₃)₂ (I)
wobei
Kt= N, P, As, Sb, S, Se
R¹, R² und R³
gleich oder verschieden
Halogen, substituiertes und/oder unsubstituiertes Alkyl CₙH₂ₙ₊₁, substituiertes und/oder unsubstituiertes Alkenyl mit 1-18 Kohlenstoffatomen und einer oder mehreren Doppelbindungen, substituiertes und/oder unsubstituiertes Alkinyl mit 1-18 Kohlenstoffatomen und einer oder mehreren Dreifachbindungen, substituiertes und/oder unsubstituiertes Cycloalkyl CₘH₂ₘ₋₁, ein- oder mehrfach substituiertes und/oder unsubstituiertes Phenyl, substituiertes und/oder unsubstituiertes Heteroaryl,
die an Kt gebundenen Gruppen können gleich oder verschieden sein
mit
n= 1-18
m= 3-7
k= 0, 1-6
y= 1-4
bedeuten.

2. Verfahren zur Herstellung von Verbindungen gemäß Anspruch 1, **dadurch gekennzeichnet, dass** ein Alkalisalz der allgemeinen Formel
D^{+ -}N(CF₃)₂ (II)
mit D⁺ ausgewählt aus der Gruppe der Alkalimetalle in einem polaren organischen Lösungsmittel mit einem Salz der allgemeinen Formel
[(R¹[CR²R³]ₖ)_{y}Kt]^{+ -}E (III)
wobei
Kt, R¹, R², R³, k und y die in Anspruch 1 angegebene Bedeutung haben und
⁻E F⁻, Cl⁻, Br⁻, I⁻, BF₄⁻, ClO₄⁻, AsF₆⁻, SbF₆⁻ oder PF₆⁻
bedeutet, umgesetzt wird.

3. Verfahren gemäß Anspruch 2 zur Herstellung der Verbindungen gemäß Anspruch 1, **dadurch gekennzeichnet, daß** Verbindungen der allgemeinen Formel
GN(CF₃)₂ (IV)
mit G ausgewählt aus der Gruppe der fluorierten Sulfonamide und fluorierten Acylamide in einem polaren organischen Lösungsmittel mit Verbindungen der allgemeinen Formel (III) mit ⁻E=F⁻ umgesetzt werden.

4. Verfahren gemäß der Ansprüche 2 und 3 zur Herstellung der Verbindungen gemäß Anspruch 1, **dadurch gekennzeichnet, daß** die Umsetzung bei Temperaturen zwischen -40 °C und 80 °C erfolgt.

5. Verfahren gemäß der Ansprüche 2 bis 4 zur Herstellung der Verbindungen gemäß Anspruch 1, **dadurch gekennzeichnet, daß** die Umsetzung in polaren organischen Lösungsmitteln ausgewählt aus der Gruppe Acetonitril, Diethoxyethan und Dimethylformamid erfolgt.

6. Verwendung der Verbindungen der allgemeinen Formel (I) als Reagenz zur Einführung von N(CF₃)₂-Gruppen in organische Verbindungen.

7. Verwendung der Verbindungen der Formel (I) bei der Synthese von Flüssigkristallverbindungen.

## Claims

1. Compounds of the general formula
[(R¹[CR²R³]ₖ)_{y}Kt]^{+ -}N(CF₃)₂ (I)
where
Kt = N, P, As, Sb, S or Se,
R¹, R² and R³
are identical or different
and are each halogen, substituted and/or unsubstituted alkyl CₙH₂ₙ₊₁, substituted and/or unsubstituted alkenyl having 1-18 carbon atoms and one or more double bonds, substituted and/or unsubstituted alkynyl having 1-18 carbon atoms and one or more triple bonds, substituted and/or unsubstituted cycloalkyl CₘH₂ₘ₋₁, mono- or polysubstituted and/or unsubstituted phenyl, substituted and/or unsubstituted heteroaryl,
the groups bonded to Kt may be identical or different,
where
n = 1-18
m = 3-7
k = 0 or 1-6
y = 1-4.

2. Process for preparing the compounds according to Claim 1, **characterized in that** an alkali metal salt of the general formula
D^{+ -}N(CF₃)₂ (II)
where D⁺ is selected from the group consisting of the alkali metals, is reacted in a polar organic solvent with a salt of the general formula
[(R¹[CR²R³]ₖ)_{y}Kt]^{+ -}E (III)
where
Kt, R¹, R², R³, k and y are as defined in Claim 1 and
⁻E is F⁻, Cl⁻, Br⁻, I⁻, BF₄⁻, ClO₄⁻, AsF₆⁻, SbF₆⁻ or PF₆⁻.

3. Process according to Claim 2 for preparing the compounds according to Claim 1, **characterized in that** compounds of the general formula
GN(CF₃)₂ (IV)
where G is selected from the group consisting of fluorinated sulfonamides and fluorinated acylamides, are reacted in a polar organic solvent with compounds of the general formula (III) where ⁻E = F⁻.

4. Process according to Claims 2 and 3 for preparing the compounds according to Claim 1, **characterized in that** the reaction is conducted at temperatures between -40°C and 80°C.

5. Process according to Claims 2 to 4 for preparing the compounds according to Claim 1, **characterized in that** the reaction is conducted in polar organic solvents selected from the group consisting of acetonitrile, diethoxyethane and dimethylformamide.

6. Use of the compounds of the general formula (I) as reagent for introducing N(CF₃)₂ groups into organic compounds.

7. Use of the compounds of formula (I) in the preparation of liquid-crystal compounds.

## Revendications

1. Composés répondant à la formule générale
[(R¹[CR²R³]ₖ)_{y}Kt]^{+ -}N(CF₃)₂ (I)
dans laquelle
Kt = N, P, As, Sb, S, Se
R¹, R² et R³
représentent simultanément ou différemment
un halogène, un alkyle en CₙH₂ₙ₊₁ substitué et/ou non substitué, un alcényle à 1-18 atomes de carbone substitué et/ou non substitué et une ou plusieurs doubles liaisons, un alcinyle à 1-18 atomes de carbone substitué et/ou non substitué et une ou plusieurs liaisons triples, un cycloalkyle en CₘH₂ₘ₋₁ substitué et/ou non substitué, un phényle mono ou polysubstitué et/ou non substitué, un hétéroaryle substitué et/ou non substitué,
les groupes liés à Kt peuvent être semblables ou différents
avec
n = 1 - 18
m = 3 - 7
k = 0, 1 - 6
y = 1 - 4.

2. Procédé de fabrication de composés selon la revendication 1, **caractérisé par le fait qu'**un sel alcalin répondant à la formule générale
D^{+ -}N(CF₃)₂ (II)
est mis à réagir avec D⁺ choisi dans le groupe des métaux alcalins dans un solvant organique polaire avec un sel répondant à la formule générale
[(R¹[CR²R³]ₖ)_{y}Kt]^{+ -}E (III)
dans laquelle
Kt, R¹, R², R³, k et y ont la même signification que dans la revendication 1 et
⁻E représente F⁻, Cl⁻, Br⁻, I⁻, BF₄⁻, ClO₄⁻, AsF₆⁻, SbF₆⁻ ou PF₆⁻.

3. Procédé de fabrication selon la revendication 2 de fabrication de composés selon la revendication 1, **caractérisé par le fait que** des composés répondant à la formule générale
GN(CF₃)₂ (IV)
avec G choisi dans le groupe des sulfonamides fluorés et des acylamides fluorés sont transformés dans un solvant polaire organique avec des composés répondant à la formule générale (III) avec ⁻E = F⁻.

4. Procédé selon les revendications 2 et 3 de fabrication de composés selon la revendication 1, **caractérisé par le fait que** la transformation s'effectue à des températures de - 40 °C à 80 °C.

5. Procédé selon les reveadications 2 à 4 de fabrication de composés selon la revendication 1, **caractérisé par le fait que** la transformation s'effectue dans des solvants organiques polaires choisis dans le groupc acctonitrilc, diéthoxyéthanc et diméthylformamide.

6. Utilisation des composés répondant à la formule générale (I) comme réactif d'introduction de groupes N(CF₃)₂ dans des composés organiques.

7. Utilisation des composés répondant à la formule (I) dans la synthèse de composés de cristaux liquides.
